# EUROPEAN PATENT APPLICATION

(11) **EP 4 098 323 A1**
(43) Date of publication of application: **07.12.2022**
(21) Application number: 21748127.4
(22) Date of filing: 25.01.2021
(51) Int. Cl.: A61P 31/04, A61K 9/02, A61K 9/06, A61K 9/08, A61K 9/14, A61K 9/70, A61F 13/15, A61F 13/20, A61F 13/511, A61L 15/32, A61L 15/44, A61K 47/06, A61K 47/38, A61K 38/40

(54) **SANITARY PRODUCT BEARING LACTOFERRIN**

(30) Priority: 30.01.2020 JP 2020013871
(71) Applicant: NRL Pharma, Inc., Takatsu-ku Kawasaki-shi, Kanagawa 213-0012 (JP); Varinos, Inc., Tokyo 135-0064 (JP)
(72) Inventor: SAKURABA, Yoshiyuki, Tokyo 141-0022 (JP); NAGAI, Yoko, Tokyo 141-0022 (JP); HOSHINO, Tatsuo, Tokyo 141-0022 (JP); OHNO, Megumi, Tokyo 141-0022 (JP)
(74) Representative: Osha Liang
(86) International application number: PCT/JP2021/002461
(87) International publication number: WO 2021/153508

(57) **Abstract**

Provided is a method for preventing the disturbance of vaginal bacterial flora during the menstrual period and improving the vaginal bacterial flora. One aspect of the present invention is a sanitary product carrying lactoferrin or a composition comprising lactoferrin and an auxiliary substance. Also provided is a kit comprising a sanitary product, and lactoferrin or the composition comprising lactoferrin and an auxiliary substance.

## Description

### TECHNICAL FIELD

The present invention relates to a sanitary product which can be used for improving the disturbance of vaginal bacterial flora during menstruation.

### BACKGROUND ART

It has been known that the use of lactoferrin as a medicine for internal or external use has an effect to improve the bacterial flora in the female reproductive organ toward a healthy state. It has been reported that by administrating a vaginal preparation of lactoferrin to a patient with refractory vaginosis, the vaginal bacterial flora turns to Lactobacillus-predominant, thereby improving a symptom (Non-Patent Documents 1, 2 and 3). On the other hand, it has been known that the vaginal bacterial flora fluctuates with the menstrual cycle (Non-Patent Document 4). Vaginal acidity may decrease due to hormonal changes just before and during menstruation and during pregnancy, thereby making the bacteria which cause an infectious disease easily proliferate. It has been reported that vaginal bacterial flora is Lactobacillus-predominant in a normal healthy condition of female at a childbearing age, but that a cycle in which the bacterial flora diversifies during menstruation and turns back to previous Lactobacillus-predominant state with the termination of menstruation is repeated (Non-Patent Document 5). The rate of turning back of the bacterial flora to its previous state varies depending on the health condition, age, and the like of each person. However, it is thought that if the bacterial flora disturbed during menstruation can be restored to its previous Lactobacillus-predominant state as quickly as possible, the risk of getting a vaginal infectious disease and the like caused by other microorganism(s) can be reduced. Further, according to a recent study, the disturbance of the bacterial flora in the female reproductive organ has been known to be one of the causes of infertility, and normalization of the bacterial flora is an important factor for normal pregnancy and delivery (Non-Patent Document 2). However, until now no report has been known on an improving agent or an improving process which is focused on the aggravation of the bacterial flora during menstruation.

### PRIOR ART DOCUMENT

### NON-PATENT DOCUMENT

Non-Patent Document 1: Pino A et al., Microbial Ecology in Health and Disease, 2017, 28, 1-11, 1357417 Bacterial biota of women with bacterial vaginosis treated with lactoferrin, open prospective randomized trial.
Non-Patent Document 2: Otsuki K. et al., Biochem. Cell Biol. 2017, 95, 31-33, Effects of lactoferrin in 6 patients with refractory bacterial vaginosis.
Non-Patent Document 3: Sessa R. et al., Biochem. Cell Biol. 2016, 00, 1-7, Effect of bovine lactoferrin on Chlamydia trachomatis infection and inflammation.
Non-Patent Document 4: Fujisawa T. et al., Bifidobacteria Microflora, 1992, 11, 1, 33-38, Effect of Menstrual Cycle and Different Age on Vaginal Microflora of Healthy Women.
Non-Patent Document 5: Hickey RJ. et al., Int. J. Obstetrics and Gynaecology, 2013, 695-706, Effects of tampons and menses on the composition and diversity of vaginal microbial communities over time.
Non-Patent Document 6: Walters, W. et al., mSystems, 2016; 1(1), e00009-15. Improved bacterial 16S rRNA gene (V4 and V4-5) and fungal internal transcribed spacer marker gene primers for microbial community surveys.
Non-Patent Document 7: Kyono K et al., Reprod Med Biol. Jul; 17(3): 297-306. Analysis of endometrial microbiota by 16S ribosomal RNA gene sequencing among infertile patients: a single-center pilot study.

### SUMMARY OF THE INVENTION

### PROBLEM TO BE SOLVED BY THE INVENTION

An object of the present invention is to provide a process for improving the disturbance of vaginal bacterial flora during menstruation.

### MEANS FOR SOLVING THE PROBLEM

The inventors of the present invention diligently studied to solve the above problem, found that a direct administration of lactoferrin into the vagina during menstruation can prevent or improve the aggravation of intravaginal bacterial flora, and have accomplished the present invention. In other words, the present invention is such that by using a sanitary product which carries lactoferrin, or a composition comprising lactoferrin and an auxiliary substance, lactoferrin which is an iron-binding protein inhibits the proliferation of iron-requiring bacteria that appear during menstruation, thereby preventing the disturbance of vaginal bacterial flora during the menstrual period and improving the vaginal bacterial flora.

According to the present invention, the followings are provided:
[1] A sanitary product carrying lactoferrin or a composition comprising lactoferrin and an auxiliary substance;
[2] The sanitary product according to the above-described [1], wherein the sanitary product is a tampon or a napkin;
[3] The sanitary product according to the above-described [1] or [2], wherein the composition is in the form of a powdery preparation, an oily liquid preparation, or an oily semi-solid preparation;
[4] The sanitary product according to any one of the above-described [1] to [3], characterized in that the amount of lactoferrin in the composition is 0.1 to 99.9% by weight based on the weight of the composition;
[5] The sanitary product according to any one of the above-described [1] to [4], characterized in that the amount of lactoferrin in the composition is 1 to 99% by weight based on the weight of the composition;
[6] The sanitary product according to any one of the above-described [1] to [5], characterized in that the amount of lactoferrin in the composition is 10 to 90% by weight based on the weight of the composition;
[7] The sanitary product according to any one of the above-described [1] to [6], characterized in that the amount of lactoferrin carried by the sanitary product is 5 to 1,000 mg/product;
[8] The sanitary product according to any one of the above-described [1] to [7], characterized in that the amount of lactoferrin carried by the sanitary product is 10 to 500 mg/product;
[9] The sanitary product according to any one of the above-described [1] to [8], characterized in that the amount of lactoferrin carried by the sanitary product is 20 to 200 mg/product;
[10] A kit comprising a sanitary product, and lactoferrin or a composition comprising lactoferrin and an auxiliary substance;
[11] The kit according to the above-described [10], wherein the sanitary product is a tampon or a napkin;
[12] A method for improving the disturbance of vaginal bacterial flora during menstruation by using the sanitary product according to the above-described [1] to [9].

### EFFECT OF THE INVENTION

With the sanitary product of the present invention, the disturbance of vaginal bacterial flora during menstruation is promptly normalized.

### BRIEF DESCRIPTION OF THE DRAWINGS

[Figure 1]
   It is a figure which shows an embodiment for preparing a sanitary product of the present invention, by including lactoferrin or a composition comprising lactoferrin and an auxiliary substance between thin layers of an absorber part of a sanitary product during a step of forming a sanitary product.
[Figure 2]
   It is a figure which shows an embodiment for preparing a sanitary product of the present invention, by including lactoferrin or a composition comprising lactoferrin and an auxiliary substance between thin layers of an absorber part of a sanitary product during a step of forming the sanitary product.
[Figure 3]
   It is a figure which shows an embodiment for preparing a sanitary product of the present invention, by impregnating a composition comprising lactoferrin and an auxiliary substance into an absorber part of a formed sanitary product by dropping or spraying the composition onto the absorber part.
[Figure 4]
   It is a figure which shows an embodiment for preparing a sanitary product of the present invention, by impregnating a composition comprising lactoferrin and an auxiliary substance into an absorber part of a formed sanitary product by dropping or spraying the composition onto the absorber part.
[Figure 5]
   It is a figure which shows an embodiment for preparing a sanitary product of the present invention, by impregnating a composition comprising lactoferrin and an auxiliary substance into an absorber of the sanitary product immediately before use of the sanitary product.
[Figure 6]
   It is a figure which shows an embodiment for preparing a sanitary product of the present invention, by impregnating a composition comprising lactoferrin and an auxiliary substance is impregnated into an absorber of the sanitary product immediately before use of the sanitary product.
[Figure 7]
   It is a figure which shows an embodiment for preparing a sanitary product of the present invention, by applying a composition comprising lactoferrin and an auxiliary substance to an absorber of the sanitary product immediately before use of the sanitary product.
[Figure 8]
   It is a figure which shows an embodiment for preparing a sanitary product of the present invention, by applying a composition comprising lactoferrin and an auxiliary substance to an absorber of the sanitary product immediately before use of the sanitary product.
[Figure 9]
   It is a figure which shows an embodiment for preparing a sanitary product of the present invention, by injecting a composition comprising lactoferrin and an auxiliary substance into a gap between an applicator which is an assisting tool for insertion of the sanitary product and an absorber immediately before use of the sanitary product.

### MODE FOR CARRYING OUT THE INVENTION

As lactoferrin which is used in the present invention, any lactoferrin can be used as long as it exhibits an improving effect on the vaginal bacterial flora. Lactoferrin is a macromolecule having a molecular weight of about 80,000 and has a property of forming a chelate with two trivalent iron ions; however, the "lactoferrin" as used in the present invention includes all types including an iron ion-free type to a completely iron-ion saturated type. Further, in the present invention, any lactoferrin can be used regardless of its origin. For example, lactoferrin derived from a living body which is extracted from milk and the like of mammals such as human being and cow and genetically modified lactoferrin produced by genetic engineering or a salt thereof can be used. Commercially available lactoferrin which is purified from milk is available from vendors such as Morinaga Milk (Japan), Tatura (Australia), Tatua (New Zealand), and the like. A process for purifying lactoferrin from milk is disclosed in detail in, for example, US Patent No. 9115211 (issued on August 25, 2015), and lactoferrin can be easily purified, basically through steps of adsorbing and desorbing lactoferrin contained in nonfat milk or whey on a cation exchange resin, followed by desalting, freeze-drying or spray drying, and the like.

In the present invention, only 1 kind of lactoferrin may be used, or 2 or more kinds of lactoferrin may be used in combination.

The sanitary product of the present invention carries lactoferrin, or a composition comprising lactoferrin and an auxiliary substance. In any case, the content of lactoferrin per 1 sanitary product can be any amount within the range in which the disturbance of vaginal bacterial flora during menstruation is speedily normalized, and can be preferably 5 to 1,000 mg/product, more preferably 10 to 500 mg/product, and particularly preferably 20 to 200 mg/product.

The amount of lactoferrin in the composition which is carried by the sanitary product of the present invention can be arbitrarily chosen within the range in which the disturbance of vaginal bacterial flora during menstruation is speedily normalized, and can be preferably 0.1 to 99.9% by weight based on the weight of the composition, more preferably 1 to 99% by weight based on the weight of the composition, and particularly preferably 10 to 90% by weight based on the weight of the composition.

As the auxiliary substance contained in the composition which is carried by the sanitary product of the present invention, an auxiliary substance in the form of powder, granule, oily solid, oily semi-solid, oily liquid, and the like can be used, and as such an auxiliary substance, ingredients or additives as exemplified below can be arbitrarily chosen and used in combination as long as the effect of the present invention is not adversely affected.

### (1) Various fats and oils

Avocado oil, almond oil, fennel oil, perilla oil, olive oil, orange oil, orange roughy oil, sesame oil, cacao butter, chamomile oil, carrot oil, cucumber oil, beef tallow fatty acid, kukui nut oil, safflower oil, shea butter, liquid shea butter, soybean oil, camellia oil, corn oil, rape seed oil, persic oil, castor oil, cotton seed oil, peanut oil, turtle oil, mink oil, egg-yolk oil, palm oil, palm kernel oil, Japan wax, coconut oil, beef tallow, lard, squalene, squalane, pristane, hydrogenated products of these fats and oils (such as hardened oil), and the like.

### (2) Waxes

Beeswax, carnauba wax, whale wax, lanolin, liquid lanolin, reduced lanolin, hard lanolin, candelilla wax, montan wax, shellac wax, rice wax, and the like.

### (3) Mineral oils

Liquid paraffine, vaseline, paraffine, ozocerite, ceresin, microcrystalline wax, and the like.

### (4) Fatty acids

Natural fatty acids such as lauric acid, myristic acid, palmitic acid, stearic acid, behenic acid, oleic acid, linolic acid, linolenic acid, docosahexaenoic acid, eicosapentaenoic acid, 12-hydroxystearic acid, undecylenic acid, tall oil, and lanolin fatty acid; and synthetic fatty acids such as isononanoic acid, caproic acid, 2-ethylbutanoic acid, isopentanoic acid, 2-methyl pentanoic acid, 2-ethyl hexanoic acid, and isopentanoic acid.

### (5) Alcohols

Natural alcohols such as ethanol, isopropanol, lauryl alcohol, cetanol, stearyl alcohol, oleyl alcohol, lanolin alcohol, cholesterol, phytosterol, and phenoxyethanol; and synthetic alcohols such as 2-hexyl decanol, isostearyl alcohol, and 2-octyl dodecanol.

### (6) Polyhydric alcohols

Ethylene oxide, ethyleneglycol, diethyleneglycol, triethyleneglycol, ethyleneglycol monoethyl ether, ethyleneglycol monobutyl ether, diethyleneglycol monomethyl ether, diethyleneglycol monoethyl ether, polyethyleneglycol, propylene oxide, propyleneglycol, polypropyleneglycol, 1,3-butyleneglycol, pentylglycol, glycerol, pentaerythritol, threitol, arabitol, xylitol, ribitol, galactitol, sorbitol, mannitol, lactitol, maltitol, and the like.

### (7) Esters

Isopropyl myristate, isopropyl palmitate, butyl stearate, hexyl laurate, myristyl myristate, oleyl oleate, decyl oleate, octyldodecyl myristate, hexyldexyl dimethyloctanoate, cetyl lactate, myristyl lactate, diethyl phthalate, dibutyl phthalate, lanolin acetate, ethyleneglycol monostearate, propyleneglycol monostearate, propyleneglycol dioleate, and the like.

### (8) Gums, saccharides, or high molecular compounds

Gum Arabic, xanthan gum, benzoin gum, dammar gum, gum guaicum, Irish moss, karaya gum, gum traganth, carob gum, quince seed, agar, casein, lactose, fructose, sucrose or esters thereof, trehalose or derivatives thereof, dextrin, gelatin, pectin, starch, carrageenan, carboxymethylchitin or chitosan, hydroxyalkyl(C2-C4)chitin or chitosan in which alkylene(C2-C4)oxide such as ethylene oxide is added, low molecular chitin or chitosan, chitosan salts, sulfated chitin or chitosan, phosphorylated chitin or chitosan, alginic acid or salts thereof, hyaluronic acid or salts thereof, chondroitin sulfate or salts thereof, heparin, ethyl cellulose, methyl cellulose, carboxymethyl cellulose, sodium carboxymethyl cellulose, carboxyethyl cellulose, sodium carboxyethyl cellulose, hydroxyethyl cellulose, hydroxypropyl cellulose, hydroxypropylmethyl cellulose, nitrocellulose, crystalline cellulose, polyethylene, polyvinyl alcohol, polyvinyl methyl ether, polyvinylpyrrolidone, polyvinyl methacrylate, polyacrylates, polyalkylene oxide such as polyethylene oxide and polypropylene oxide or crosslinked polymers thereof, carboxyvinyl polymer, polyethyleneimine, silicone, polysiloxane, and the like.

As described above, while the composition which is carried by the sanitary product of the present invention can comprise lactoferrin and an auxiliary substance, various solvents, preservatives, solubilizing agents, stabilizers and the like which are usually used in pharmaceuticals or cosmetics can be further arbitrarily added as necessary in an amount within the range in which the disturbance of vaginal bacterial flora during menstruation is speedily normalized. Further, another pharmaceutical agent can be added as necessary.

The composition which is carried by the sanitary product of the present invention is preferably in a form which is convenient for intravaginal administration, for example, a powdery preparation, an oily liquid preparation, or an oily semi-solid preparation, and can be prepared by, e.g., mixing or stirring the above described lactoferrin and an auxiliary substance. Since lactoferrin is unstable at a high temperature or a high humidity, the composition is preferably prepared under a dry condition.

The sanitary product of the present invention can be a sanitary product having an arbitrary form which enables the delivery of lactoferrin into the vagina, and a tampon and a napkin can be exemplified. In the case where the sanitary product of the present invention is a tampon or a napkin, the content of lactoferrin can be an arbitrary amount within the range in which the disturbance of vaginal bacterial flora during menstruation is speedily normalized, and can be preferably 5 to 1,000 mg/product, more preferably 10 to 500 mg/product, and particularly preferably 20 to 200 mg/product.

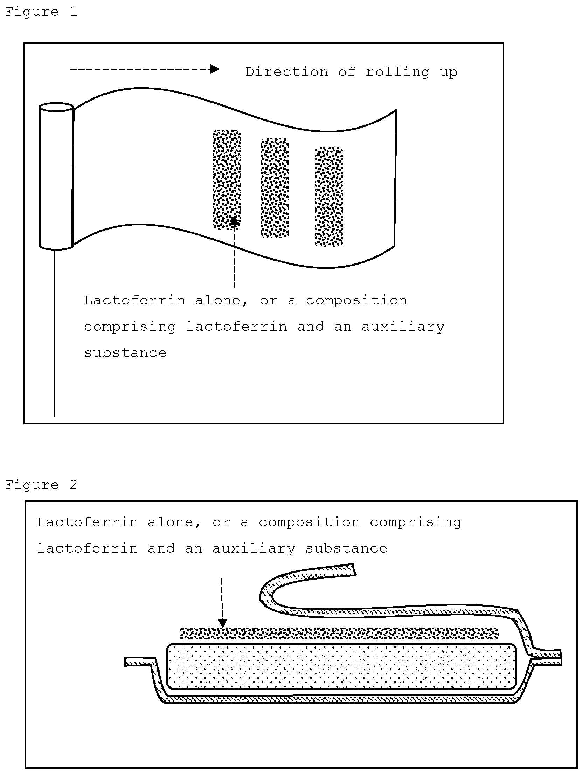

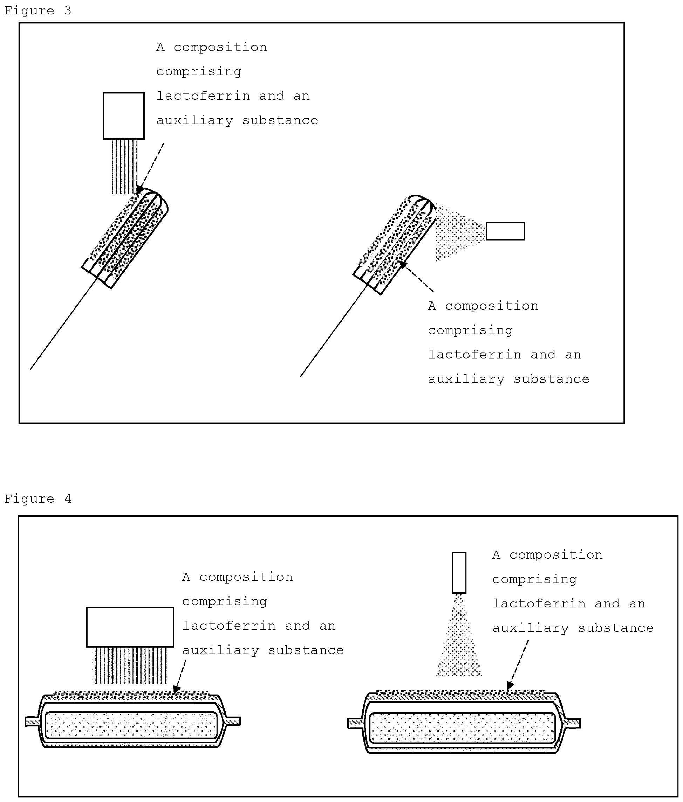

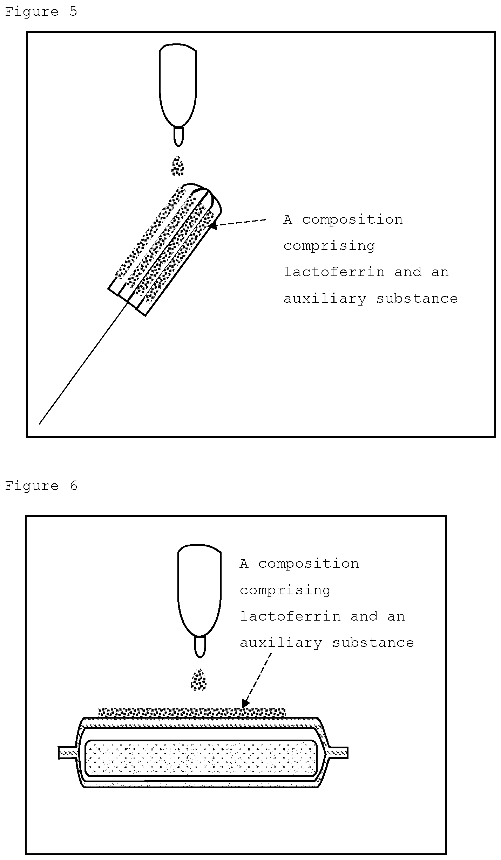

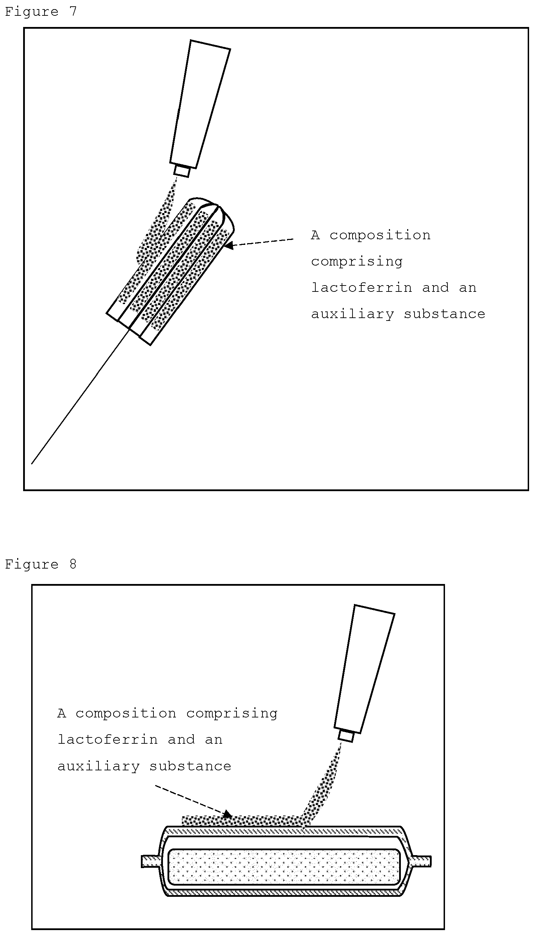

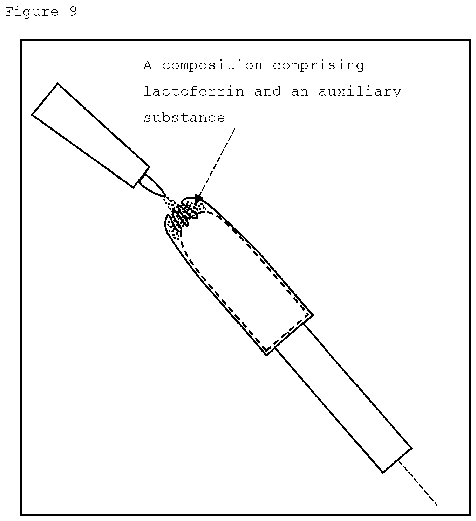

The sanitary product of the present invention can be prepared by making a sanitary product such as a tampon or a napkin to carry lactoferrin or a composition comprising lactoferrin and an auxiliary substance. As a process for carrying, the following processes can be exemplified. The following processes are for explanation of the present invention, and do not limit the present invention at all.
1. Lactoferrin alone, for example, 0.2 g of lactoferrin is airtightly included between thin layers of an absorber part of a sanitary product during a step of forming the sanitary product. (Figure 1) (Figure 2)
2. A composition comprising lactoferrin and an auxiliary substance, for example, 0.1 g of a powdery preparation comprising 90% by weight of lactoferrin is airtightly included between thin layers of an absorber part of a sanitary product during a step of forming the sanitary product. (Figure 1) (Figure 2)
3. A composition comprising lactoferrin and an auxiliary substance, for example, 0.5 g of an oily liquid preparation comprising 20% by weight of lactoferrin is water-tightly included between thin layers of an absorber part of a sanitary product during a step of forming the sanitary product. (Figure 1) (Figure 2)
4. A composition comprising lactoferrin and an auxiliary substance, for example, 1 g of an oily liquid preparation comprising 10% by weight of lactoferrin is impregnated by dropping or spraying it onto an absorber part of a formed sanitary product. (Figure 3) (Figure 4)
5. A composition comprising lactoferrin and an auxiliary substance, for example, 0.2 g of an oily liquid preparation comprising 20% by weight of lactoferrin is impregnated into an absorber immediately before use of the sanitary product. (Figure 5) (Figure 6)
6. A composition comprising lactoferrin and an auxiliary substance, for example, 0.4 g of an oily semi-solid preparation comprising 10% by weight of lactoferrin is applied to an absorber immediately before use of the sanitary product. (Figure 7) (Figure 8)
7. A composition comprising lactoferrin and an auxiliary substance, for example, 0.2 g of an oily liquid preparation or an oily semi-solid preparation comprising 20% by weight of lactoferrin is injected into a gap between an applicator which is an assisting tool for insertion of a sanitary product and an absorber immediately before use of the sanitary product. (Figure 9)

The sanitary product of the present invention which carries lactoferrin or a composition comprising lactoferrin and an auxiliary substance can be used depending on the condition of menstruation. For example, 4 to 5 tampons per day or 7 to 18 napkins per day can be used throughout the menstrual period.

### EXAMPLES

Hereinafter, the present invention will be more specifically described by way of Examples.

### Example 1

### <Composition comprising lactoferrin and an auxiliary substance>

Various compositions that were carried by the sanitary product of the present invention were prepared. The formulation examples are shown below, but since the compositions were prepared by a process of mixing and stirring, only the amounts are shown.

Appropriate amounts of lactoferrin (produced by Tatura Co., Australia) and an auxiliary substance(s) (various oils and fats, waxes, mineral oils, fatty acids, alcohols, polyhydric alcohols, esters, gums, saccharides, or high molecular compounds, and the like) were mixed. Lactoferrin was in the range of 0.1 to 90% by weight. Specific formulation examples are shown as follows.

### (Formulation example 1) Oily semi-solid preparation

| | |
|---|---|
| Lactoferrin | 10% |
| Liquid paraffin | 75% |
| Sodium carboxymethyl cellulose | 8% |
| Polyethylene | 6% |
| Pectin | 1% |

### (Formulation example 2) Oily semi-solid preparation

| | |
|---|---|
| Lactoferrin | 40% |
| Vaseline | 60% |

### (Formulation example 3) Oily liquid preparation

| | |
|---|---|
| Lactoferrin | 0.1% |
| Liquid paraffine | 99.9% |

### (Formulation example 4) Oily liquid preparation

| | |
|---|---|
| Lactoferrin | 20% |
| Butyleneglycol | 8% |
| Glycerol | 72% |

### (Formulation example 5) Oily liquid preparation

| | |
|---|---|
| Lactoferrin | 30% |
| Squalane | 70% |

### (Formulation example 6) Powdery preparation

| | |
|---|---|
| Lactoferrin | 80% |
| Hydroxypropylmethyl cellulose | 20% |

### (Formulation example 7) Powdery preparation

| | |
|---|---|
| Lactoferrin | 90% |
| Xanthan gum | 10% |

### Example 2

### <Carrying of lactoferrin or a composition comprising lactoferrin and an auxiliary substance on a sanitary product>

### (Lactoferrin)

Lactoferrin (Tatura Co., Australia), 0.1 g, was added between the thin layers of an absorber part of a tampon during a step of forming the tampon, then the absorber part was formed into a cylindrical shape and compressed, thus obtaining the tampon of the present invention. (Figure 1)

### (Lactoferrin)

Lactoferrin (Tatura Co., Australia), 0.2 g, was added between thin layers of an absorber part of a napkin during a step of forming the napkin, then the napkin was formed to obtain the napkin of the present invention. (Figure 2)

### (Oily liquid preparation)

Lactoferrin (Tatura Co., Australia), 20 g, was added to 80 g of squalane, followed by stirring to prepare a composition. The composition, 0.5 g, was dropped or sprayed onto an absorber part of a tampon which had been formed, thus obtaining the tampon of the present invention. (Figure 3)

### (Oily liquid preparation)

Lactoferrin (Tatura Co., Australia), 10 g, was added to 90 g of liquid paraffin, followed by stirring to prepare a composition. The composition, 2 g, was dropped or sprayed onto an absorber part of a napkin which had been formed, thus obtaining the napkin of the present invention. (Figure 4)

### (Oily semi-solid preparation)

10 g of lactoferrin (Tatura Co., Australia), 8 g of cellulose gum, 6 g of polyethylene, and 1 g of pectin were added to 75 g of liquid paraffin, followed by mixing to prepare a composition. The composition, 0.38 g, was injected into a gap between an applicator and an absorber of a tampon (an applicator was attached) immediately before use of the tampon to obtain the tampon of the present invention. (Figure 9)

### EXAMPLE 3

### CLINICAL STUDY

In the clinical study, for 9 weeks including 3 menstrual cycles in subjects who had agreed with the research, a method in which the vaginal bacterial flora was compared before and after the use of lactoferrin-injected tampon within the same menstrual cycle was employed.

The lactoferrin-injected tampon was prepared by injecting 0.38 g of the oily semi-solid preparation of Formulation example 1 comprising 10% by weight of lactoferrin (Tatura Co., Australia) (corresponding to 38 mg of lactoferrin) into the gap between the applicator and the absorber of Sofy Soft Tampon Regular (Unicharm Co.) .

The subjects started the use of the lactoferrin-injected tampons from the 2nd or 3rd menstrual cycle, and changed the tampon every 4 hours.

The sampling method of the specimens was such that each subject collected the specimens by using the swab brushs of an intravaginal flora DNA sampling kit (product name: "OMNIgene VAGINAL" (DNA Genotek Inc., Canada)) by herself. The collected vaginal mucus was transferred into 1 ml of a preservation liquid (product name: "MMB collection tube" (DNA Genotek Inc., Canada)) for inactivation and stabilization of the bacteria and stored at an ordinary temperature.

Subject 1 avoided the intake of lactic acid bacteria-containing foods, fermented foods and the like to the utmost from the start of the first menstruation which was before the intervention, and the specimen was collected on the 7th day after the start of menstruation. After that, Subject 1 used the lactoferrin-injected tampons during the 2nd menstruation cycle and the 3rd menstruation cycle, and the respective specimen was collected on the 7th day after the start of menstruation. As a result, by using the lactoferrin-injected tampons, an increase in the vaginal Lactobacillus % was found in the 3rd menstrual cycle (Table 1).

**Table 1. Vaginal Lactobacillus % in Subject 1**

| | Whether the tampons were used in the latest menstruation or not | Vaginal Lactobacillus % |
|---|---|---|
| 7th day of the 1st cycle | Not used | 35.7% |
| 7th day of the 2nd cycle | Used | 37.7% |
| 7th day of the 3rd cycle | Used | 61.2% |

In Subject 2, the long-term effect was further investigated. Subject 2 used the lactoferrin-injected tampons from the 1st cycle of menstruation, and the specimens were collected for 3 cycles on the 7th day after the start of menstruation. As a result, by using the lactoferrin-injected tampons for 3 cycles, an increase in the vaginal Lactobacillus % was found (Table 2).

**Table 2. Vaginal Lactobacillus % in Subject 2**

| | Whether the tampons were used in the latest menstruation or not | Vaginal Lactobacillus % |
|---|---|---|
| 7th day of the 1st cycle | Used | 81.5% |
| 7th day of the 2nd cycle | Used | 95.7% |
| 7th day of the 3rd cycle | Used | 98 .1% |

In Example 3, the vaginal bacterial flora analysis was carried out as follows.

The vaginal mucus which had been collected by the subject herself was transferred into 1 ml of a preservation liquid (product name: "MMB collection tube" (DNA Genotek Inc., Canada)) for inactivation and stabilization of the bacteria. In order to extract the bacterial genome DNA, proteinase K and lysozyme were added to the preservation liquid to dissolve the bacteria. The genome DNA was extracted by using a DNA extraction kit (product name: "Agencourt Genfind v2 Blood & Serum DNA Isolation Kit" (Beckman Coulter Inc. USA)). The concentration of the extracted DNA was determined by using a kit of the product name "Qubit dsDNA HS Assay Kit" (ThermoFisher Scientific K.K.)

Bacterial flora analysis was carried out by a 16S amplicon sequence method using a next generation sequencer. Based on the protocol of "Earth Microbiome Project" (Non-Patent Document 6), the bacterial DNA was amplified by using primers which had been ligated with a sequence for amplifying the V4 region of 16S rRNA gene and the Illumina Nextera XT adaptor sequence (Non-Patent Document 7). A mixture containing 25 ng/ µL of DNA, each 200 µmol/L of deoxyribonucleotide triphosphates, 400 nmol/L of each primer, 2.5 U of product name: "FastStart Hifi polymerase", 20 mg/mL of BSA (Sigma), 0.5 mol/L of betaine (Sigma), and MgCl₂-containing buffer (Roche) was prepared for PCR. The PCR was carried out by using a thermal cycler (product name: "SimpliAmp Thermal Cycler" (Thermo Fisher)) in which degeneration at 94°C for 2 minutes, followed by 30 cycles of 94°C for 20 seconds, 50°C for 30 seconds, and 72°C for 1 minute, and finally a reaction at 72°C for 5 minutes were carried out. The PCR product was purified with a product named "A gencourt AMPure XP" (Beckman Coulter Inc, USA). Then, a library was prepared by using a kit of the product name "Nextera XT Index kit" (Illumina Inc., USA) based on "llumina 16S Metagenomic Sequencing Library Preparation protocol" (https://support.illumina.com/documents/documentation/chemistry_doc umentation/16s/16s-metagenomic-library-prep-guide-15044223-b.pdf). The sequence of the prepared library was determined with pair end sequence of 2x200-bp by using a kit of the product name "MiSeq Reagent Kit v3" (Illumina K.K.). For the data analysis, the quality check of whole sequence was carried out by using "fastqQC" (https://www.bioinformatics.babraham.ac.uk/projects/fastqc/), and the bacteria were identified by using "USEARCH" (https://www.drive5.com/usearch/) and "QIIME" (http://qiime.org/) at their genus levels.

The present application is based on the Japanese Patent Application No. 2020-013871 filed on January 30, 2020, and the subject matters described in the specification and the scope of the claims for patent of Japanese Patent Application No. 2020-013871 are all incorporated in this specification.

## Claims

1. A sanitary product carrying lactoferrin or a composition comprising lactoferrin and an auxiliary substance.

2. The sanitary product according to Claim 1, wherein the sanitary product is a tampon or a napkin.

3. The sanitary product according to Claim 1 or 2, wherein the composition is in the form of a powdery preparation, an oily liquid preparation, or an oily semi-solid preparation.

4. The sanitary product according to any one of Claims 1 to 3, **characterized in that** the amount of lactoferrin in the composition is 0.1 to 99.9% by weight based on the weight of the composition.

5. The sanitary product according to any one of Claims 1 to 4, **characterized in that** the amount of lactoferrin in the composition is 1 to 99% by weight based on the weight of the composition.

6. The sanitary product according to any one of Claims 1 to 5, **characterized in that** the amount of lactoferrin in the composition is 10 to 90% by weight based on the weight of the composition.

7. The sanitary product according to any one of Claims 1 to 6, **characterized in that** the amount of lactoferrin carried by the sanitary product is 5 to 1,000 mg/product.

8. The sanitary product according to any one of Claims 1 to 7, **characterized in that** the amount of lactoferrin carried by the sanitary product is 10 to 500 mg/product.

9. The sanitary product according to any one of Claims 1 to 8, **characterized in that** the amount of lactoferrin carried by the sanitary product is 20 to 200 mg/product.

10. A kit comprising a sanitary product, and lactoferrin or a composition comprising lactoferrin and an auxiliary substance.

11. The kit according to Claim 10, wherein the sanitary product is a tampon or a napkin.

12. A method for improving the disturbance of vaginal bacterial flora during menstruation by using the sanitary product according to Claims 1 to 9.
